# EUROPEAN PATENT SPECIFICATION

(11) **EP 3 860 505 B1**
(45) Date of publication and mention of the grant of the patent: **18.02.2026**
(21) Application number: 19765979.0
(22) Date of filing: 04.09.2019
(51) Int. Cl.: A61F 2/01, A61F 2/24, A61B 17/221, A61F 2/966

(54) **PROTECTIVE DEVICE FOR OPEN HEART AORTIC VALVE REPLACEMENT SURGERY AND KIT COMPRISING THE SAME**
SCHUTZVORRICHTUNG FÜR OFFENE HERZKLAPPENERSATZCHIRURGIE UND KIT DAMIT
DISPOSITIF DE PROTECTION DESTINÉ À UNE CHIRURGIE DE REMPLACEMENT DE VALVULE AORTIQUE À COEUR OUVERT ET KIT LE COMPRENANT

(30) Priority: 04.10.2018 EP 18198605
(43) Date of publication of application: 11.08.2021
(73) Proprietor: Albert-Ludwigs-Universität Freiburg, 79085 Freiburg (DE)
(72) Inventor: SHAHINIAN, Jasmin, 4052 Basel (CH)
(74) Representative: Rösler, Uwe
(86) International application number: PCT/EP2019/073619
(87) International publication number: WO 2020/069810

(56) References cited:
- EP-A1- 1 408 873
- EP-B1- 1 408 873
- WO-A1-2017/157884

## Description

### Field of the Invention

The present invention generally relates to a protective device for open heart aortic valve replacement surgery. In particular, the device is intended for collecting and disposing any calcified fragments and tissue remnants falling off as debris during removal of a sclerotic valve.

### Background of the Invention

Aortic valve replacement is a procedure applied when a patient's aortic valve is failing, which can be due to various causes. The valve can either become leaky, a condition called aortic insufficiency, or it can be partially blocked in a condition called aortic stenosis. Current approaches for aortic valve replacement include open heart surgery via a sternotomy, minimally invasive cardiac surgery and transcatheter aortic valve replacement.

Open heart aortic valve replacement is usually done through a median sternotomy, i.e. incision is made by cutting through the sternum. After the pericardium has been opened, the patient is connected to a cardiopulmonary bypass machine which takes over the tasks of breathing and pumping around the patient's blood around while the damaged heart valve is being replaced. Once the bypass is functional, a cross clamp is applied, followed by administration of cardioplegic solution/blood-cardioplegia either via aorta ascendens and/or selectively via coronary ostiae. After aortotomy is performed, the surgeon removes the patient's diseased aortic valve and replaces it with a suitable mechanical or tissue valve. After the valve is in place, the aorta can be closed and the patient can be taken off the cardiopulmonary bypass machine.

A complication that can arise in aortic valve replacement surgery is caused by small calcified fragments falling off as debris during sclerotic valve removal e.g. by using a Rongeur tool. Such fragments and debris can pass into the left ventricle and thus pose a significant risk of post-surgical stroke.

Embolic protection devices are known in the field of interventional cardiology, but most of them are inserted in the bloodstream where they act as an implanted sieve.

DE 10049812 A1 discloses a filter type device for removing macroscopic particles from the bloodstream of a patient undergoing minimally invasive aortic valve surgery. The known device comprises a pair of screen elements that can be unfolded in umbrella-type manner so as to form a contained volume therebetween. For the aortic valve intervention, the device is positioned in such manner that the valve is enclosed between the two screen elements. Accordingly, any macroscopic debris formed during intervention on the aortic valve will be safely trapped in the contained volume. However, the principle of this known device is not suitable for open heart aortic valve surgery which requires that access to the valve is not hindered by a screen element or the like.

US 2005/119688 A1 discloses a method and assembly for distal embolic protection following open heart aortic valve replacement surgery. US 2017/071720 A1 discloses an apparatus and procedure for trapping embolic debris. US 2002/161394 A1 discloses an aortic filter catheter. All of these previously described devices are based on the filter principle already mentioned above. In other words, they are configured in such manner as to trap any debris while allowing for an arterial blood flow which should be as unhindered as possible.

EP 1 408 873 A1 discloses a delivery catheter for delivering and deploying an embolic protection filter to a desired site in a vasculature, said catheter is configured to facilitate rapid exchange over a guidewire during both delivery and deployment of the filter. The embolic protection filter is arranged slideable along a section of a guidewire and radial expandable after separating from an delivery catheter which otherwise encompass the filter during delivery and withdrawal.

In view of the above, there is still a need to provide an effective embolic protection device that could be efficiently and safely implemented in currently used open heart aortic valve replacement surgery.

### Summary of the Invention

The above and other objects are achieved by the present protective inventive device as defined by independent claim 1.

In the present context, the terms "proximal" and "distal" will be understood as directed towards and away, respectively, from an operator holding the device.

The use of the device in open heart aortic valve replacement surgery starts after the damaged valve has been exposed by making a cut in the aorta and applying a cross-clamp. The device is initially in a movable state, i.e. in a state in which the the strut arrangement is longitudinally expanded. In this longitudinally expanded state, the tip element is axially displaced from the base element. Accordingly, the strut arrangement has a correspondingly reduced radial size, analogous to a folded umbrella.

At this point of the procedure, the third coupling member and the fifth coupling member are in a connected state, i.e. the distal end of the elongated actuator member and the tip element are connected to each other. Moreover, the handling member is connected to the base element so as to facilitate the positioning of the protective device in the aorta.

The device is then driven with its tip region through the damaged aortic valve and positioned in such manner that (i) the tip element is located distally from the aortic annulus and points into the left ventricle and (ii) the base element is located just slightly distally from the aortic annulus. This procedure evidently requires that the distal part of the device that is driven through the damaged aortic valve is sufficiently narrow. For interventions in adults, the distal part of the device should have an outer diameter not exceeding about 5 mm.

After positioning, the device is brought to a deployed state by retracting the tip element in proximal direction by a corresponding retraction of the actuator member connected thereto. This retraction is continued until the tip element meets the base element, whereby the fourth coupling member, which is disposed on the tip element, is connected with the second coupling member disposed on the base element. As a consequence of this retraction, the strut arrangement is radially expanded so as to form an anchoring support for attachment to a surrounding arterial wall segment, i.e. the aortic annulus. The radial expansion of the strut arrangement leads to unfolding of the screen element surroundingly attached thereto, analogous to opening an umbrella. As a result, the unfolded screen element is now fixed in a position below the aortic valve.

Next, the connection between the third coupling member and the fifth coupling member is released, i.e. the distal end of the actuator member is disengaged from the tip element. This enables retracting, i.e. pulling away the actuator member so as to clear the central region of the aortic valve. Moreover, the connection between the sixth coupling member and the first coupling member is also disconnected, so as to allow retracting the handling member and thus also clear the peripheral region of the aortic valve. The deployed state of the device is maintained by virtue of the connection between the fourth and second coupling members.

Subsequently the diseased aortic valve is removed by an appropriate surgical technique. Any debris formed during this intervention that falls downwards from the valve region is caught by the unfolded screen element.

Once the aortic valve has been removed, the device is removed in the following manner. First, the handling member is reconnected to the base element by bringing together the sixth and the first coupling member. Second, the elongated actuator member is reinserted through the longitudinal passageway of the handling member. The distal end of the actuator member is advanced until it meets the tip element. At this point the third coupling member is re-connected to the fifth coupling member of the tip element. Thereafter, the tip element is disengaged from the base element by releasing the connection between the fourth and second coupling elements. Subsequently, the elongated actuator member is advanced further, thereby causing an axial expansion and a corresponding radial collapse of the strut arrangement and screen element. The device thus reaches again its movable state and is ready to be retracted in its entirety. By the refolding of the screen element it is ensured that its peripheral portion does not engage in the aortic wall and that no debris falls off from the screen element. Finally, an appropriate replacement valve, which can be a mechanical or tissue valve, is put in place.

It will be understood that the entire procedure needs to be conducted in accordance with standard requirements for surgery. Therefore, according to a second aspect of the invention (claim 18), a kit for open heart aortic valve replacement surgery comprises a protective device as defined above which is provided in a sterilized packaged form.

Preferred embodiments of the invention are defined in the dependent claims and/or are described hereinbelow.

In view of its function of collecting debris falling off during sclerotic valve removal in open heart surgery, there is no need to ensure an uninterrupted blood passage. In simple words, the screen element does not work like a filter or sieve, but rather like a closed trap. Accordingly, the screen element can be made from any suitable thin and flexible material, wherein "suitable" refers to general compatibility with surgical interventions. In one embodiment, the screen element is made from a thin polymeric or metallic mesh. In other embodiments, the screen element is made of a non-porous foil or cloth.

In principle, various embodiments can be contemplated for the various coupling elements serving to form releasable connections between parts of the device. Unless explicitly mentioned otherwise, the term "releasable connections" shall be understood in the sense of a repeatedly connectable and releasable connection. Depending on specific function and configuration of the involved parts, different types of couplings such as threaded connections, snap connections or bayonet-type connections are appropriate.

According to one embodiment, the second coupling member and the fourth coupling member are configured as cooperating snap connections forming the intended releasable connection between the tip element and the base element. Such a snap connection generally requires applying, in an appropriate direction, an axial force that exceeds a predefined threshold for being brought into a connected or disconnected state, respectively. This threshold needs to be sufficiently high to ensure that the strut arrangement is maintained in its radially expanded state, but it also needs to be sufficiently low to enable a safe release of the tip element after completion of the aortic valve removal without requiring undue force. Alternatively or additionally, the radially expanded state is reached and maintained by means of a fluid actuated expandable structure. Such fluid actuation may be achieved pneumatically or hydraulically by appropriate fluid delivery means.

According to another embodiment, the first coupling member and the sixth coupling member are configured as cooperating threads, i.e. as a first cooperating thread pair, thereby forming a threaded releasable connection between the distal end of the elongated handling member and the proximal side of the base element. Advantageously, the elongated handling member is configured as a metallic tube with a smooth external wall, and the sixth coupling member is formed thereon as an internal thread. A corresponding external thread is then provided on the base element, at the proximal side thereof. Connecting and disconnecting is then achieved by appropriate rotation. In particular, if the base element is held in place, e.g. when fixed to an arterial wall region, such rotation is simply effected on the elongated handling member.

According to a further embodiment, the third coupling member and the fifth coupling member are configured as a second pair of cooperating threads, thereby forming a threaded releasable connection between the distal end of the elongated actuator member and the tip element. Advantageously, the elongated actuator is configured as a thin metallic rod, and the third coupling member is formed thereon as an external thread. A corresponding internal thread is then provided on the tip element, at the proximal side thereof. Connecting and disconnecting is achieved by appropriate rotation. In particular, if the base element is held in place, e.g. when fixed to an arterial wall region, such rotation is simply effected on the elongated actuator member.

According to an advantageous embodiment, the first cooperating threads and the second cooperating threads have identical sense of rotation and, moreover, the elongated actuator member and the elongated handling member are rotationally coupled but longitudinally decoupled. On the one hand, rotational coupling implies that by applying a single rotational motion an operator can simultaneously rotate the actuator member and the handling member, thereby connecting or disconnecting both pairs of cooperating threads. Longitudinal decoupling, on the other hand, allows compensation for different pitches of the two pairs of cooperating threads. In certain embodiments, rotational coupling with longitudinal decoupling is achieved by means of a radially oriented pin or stick which is firmly connected to one member and is engaged in a corresponding axial slit or recess of the other member.

Appropriate positioning of the device along the aortic section of interest is of crucial importance for ensuring proper functioning. In particular, the device shall be positioned in such manner that the strut arrangement in its radially expanded state meets a mechanically stable region of the aorta between the aortic valve and the left ventricle. Therefore, according to yet another embodiment, the handle member comprises a radially protruding structure configured to define an operational longitudinal position of the device. In some embodiments, the radially protruding structure is configured as a collar formed on a substantially cylindrical wall region. In other embodiments, the radially protruding structure is formed by a step-like increase in radial direction.

In accordance with the present invention, the protective device further comprises a longitudinally retractable sheath element configured to surround the screen element when the device is in its folded state. Such a sheath element acts as a mechanical protection for the device. Moreover, it can be configured with a rounded edge region allowing for easy passage through the aortic valve to be replaced. In order to allow retraction of the sheath element in embodiments provided with a radially protruding structure, the latter can be provided with longitudinal passages formed as slits, e.g. as segments of a circle. Such passages are intended to receive a wall region of the sheath element which is correspondingly configured as longitudinal stripes or ribs.

For the necessary unfolding and refolding of the screen element, it is part of the invention that the flexible strut arrangement comprises a plurality of resilient struts, each one having a proximal strut end connected to a radial face portion of the base element and a distal strut end connected to a radial face portion of the tip element.

However, the term "flexible strut arrangement" shall not imply that the arrangement is necessarily made up from resilient struts. Alternatively, in accordance with the present invention, the flexible strut arrangement comprises a plurality of articulated struts, each articulated strut comprising at least two strut segments connected to each other by a sliding hinge member. Also in this case, each articulated strut has a proximal strut end connected to a radial face portion of the base element and a distal strut end connected to a radial face portion of the tip element. The term "sliding hinge member" shall be understood as a mechanical coupling member which allows for simultaneous sliding and pivoting motion of two connected strut segments. More specifically, an end of one strut segments is allowed to slide along the other segment, and the angle between the two strut segments about a pivot axis of the sliding hinge member is allowed to change.

According to one embodiment, the resilient struts are connected to the base element and to the tip element in a hinged manner.

According to an advantageous embodiment of the base element, the latter comprises:
- a distal base ring provided with said radial face portion and said second coupling member, said face portion having a plurality of lateral connection passages for receiving respective proximal strut ends,
- a covering ring having a flange region and a protruding cylindrical collar provided with longitudinal slits forming passages for axially sliding the covering ring over said proximal strut ends,
- a proximal coupling ferrule having a distal cylindrical part insertable through the covering ring and connectable to the distal base ring, the coupling ferrule being provided with said first coupling member and having a radially protruding section for abutment against a proximal surface of the covering ring,
thereby providing an easily assemblable, compact construction.

Advantageously, the screen element comprises an expandable ring element connected to the strut arrangement and forming a radially external portion of the screen element. Such a ring element can act as a cushion-like structure ensuring safe anchoring of the device against a surrounding arterial wall region. According to one embodiment, the ring element can be pneumatically or hydraulically actuatable and thereby provide or assist the unfolding/refolding of the screen element.

Various further embodiments take into account the intended application of the protective device for open heart aortic valve replacement surgery. Generally, the device can be provided in somewhat different sizes, which are selected primarily according to the currently known size ranges of the aortic valve annulus.

According to one embodiment, in the movable state of the device, a distal part thereof has an outer diameter not exceeding about 5 mm. This allows the intended insertion through the aortic valve to be replaced. Depending on the specific configuration of the device, this limiting outer diameter is defined by a retractable sheath element or by the radial extension of the strut arrangement in its longitudinally extended state.

According to another embodiment, in the deployed state of the device, the screen element in its unfolded state has an outer diameter of 20 to 35 mm. This should substantially correspond to the inner diameter of the aortic region against which the device shall be anchored during the surgical intervention.

According to yet another embodiment, the device further comprises a longitudinal marker scale on the elongated handling member and/or on the sheath element. The marker scale is intended as a visual positioning aid.

According to a further embodiment, the device further comprises at least two string elements, each one of said string elements being connected at its distal string end to a respective connection point of the screen element and having a length sufficient for pulling a proximal string end past the base element. In this manner, when the device is in its deployed state and the elongated handling member has been removed, the string element can be grabbed at its distal end and pulled upwards past the base element and secured by the operator for the case of later need. In particular, by applying a pulling action to the string elements when the device is in its deployed state, it is possible to reduce the radial extension of the strut arrangement and thereby disengage the device from the surrounding aortic region. This allows removing the device also in situations where the generally intended reconnection procedure has failed.

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein are shown:
- Fig. 1: a first embodiment of a protective device, in the movable state, partially disassembled, in a schematic view as a longitudinal section;
- Fig. 2: the protective device of Fig. 1, in the deployed state;
- Fig. 3: the device of Fig. 3, with an attached sheath element;
- Fig. 4: a second embodiment of a protective device, in the movable state, in a schematic view as a longitudinal section;
- Fig. 5: a third embodiment of a protective device, in the movable state with attached sheath element, in a schematic view as a longitudinal section;
- Fig. 6: the protective device of Fig. 5, in the deployed state;
- Fig. 7: the protective device of Fig. 5, shown in an inserted position abutting against an aortic valve;
- Fig. 8: the protective device of Fig. 7, in the deployed state;
- Fig. 9: a photographic model view of a distal part of a protective device, in the deployed state;
- Fig. 10: a photographic model view of a protective device, in a transition state between movable and deployed states;
- Fig. 11: a distal part of a fourth embodiment of a protective device, in the movable state, in a perspective view;
- Fig. 12: the distal part of Fig. 11, in a partially disassembled state, in a magnified view;
- Fig. 13: the distal part of Fig. 11, in a sectional view;
- Fig. 14: a screen element of the protective device, in a perspective view;
- Fig. 15: another embodiment of the the distal part of Fig. 11, in a sectional view;
- Fig. 16: a fifth embodiment of a protective device, in the movable state, in a schematic view as a longitudinal section; and
- Fig. 17: a sixth embodiment of a protective device, in the movable state, in a perspective view.

### Detailed description

It will be understood that the figures are not necessarily drawn to scale. In some instances, relative dimensions are substantially distorted for ease of visualization. Features having identical purpose will generally be denoted with identical reference numerals also for different embodiments.

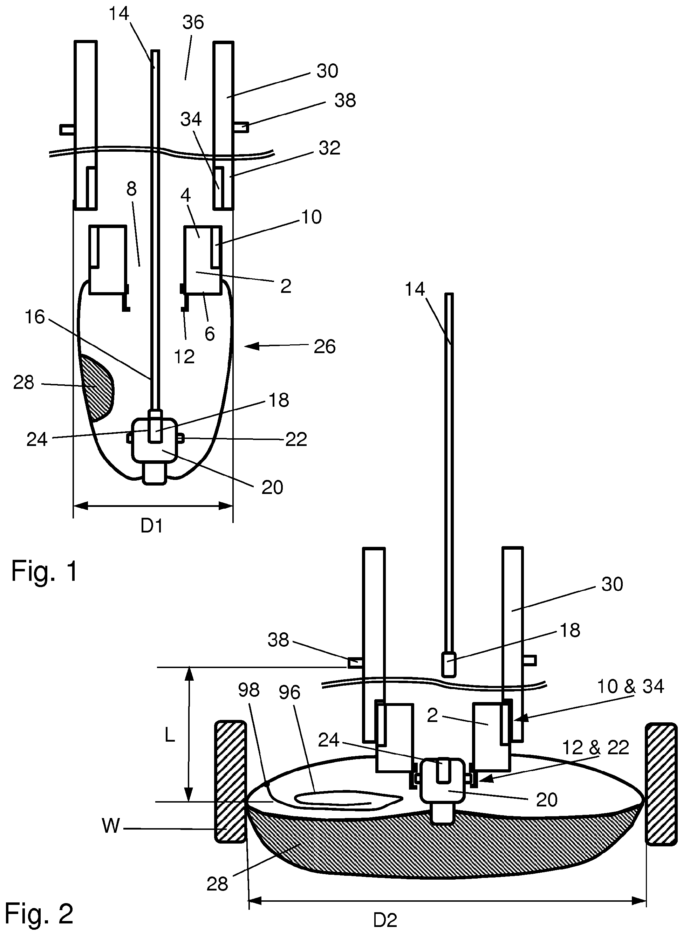

As shown schematically in Figs. 1 and 2, a protective device for open heart aortic valve replacement surgery, comprises a base element 2 having a proximal side 4, a distal side 6 and a central passage 8 between the proximal side 4 and the distal side 6. The base element 2 further comprises, on its proximal side 4, a first coupling member 10 configured as an external thread and, on its distal side 6, a second coupling member 12 configured as a snap connection element. The device further comprises an elongated actuator member 14 which is longitudinally slidable within the central passage 8 and has a distal end 16 provided with a third coupling member 18. In the example shown, the third coupling member 18 is configured as a terminal external thread. A tip element 20 of the device comprises a fourth coupling member 22, which in the example shown is configured as a snap element that is releasably connectable with the second coupling member 12. Moreover, the tip element 20 comprises a fifth coupling member 24 configured as a proximally situated internal thread which is releasably connectable with the third coupling member 18 of the actuator member 14.

The protective device also comprises a flexible strut arrangement 26 forming a radially external connection between the base element 2 and the tip element 20 and a screen element 28 surroundingly attached to the strut arrangement 26. In the example shown, the screen element 28 is made of a non-porous foil or cloth. Moreover, the device comprises an elongated handling member 30 having a distal end 32 provided with a sixth coupling member 34 configured as an inner thread which is releasably connectable with the first coupling member 10 of the base element 2. In general, the handling member 30 has a longitudinal passageway 36 configured to slidingly receive the actuator member 14. In the schematic example of Figs. 1 and 2 the longitudinal passageway 36 is constituted by the inside of a tubular shaped handling member 30, which in practice will generally be longer than shown in the figures.

In a deployed state of the device as shown in Fig. 2, the fourth coupling member 22 is connected with the second coupling member 12 and the strut arrangement 26 is radially expanded, thereby forming an anchoring support for attachment to a surrounding arterial wall segment W. In a movable state of the device as shown in Fig. 1 with partially disassembled components, the fourth coupling member 22 is released from the second coupling member 12 and the tip element 20 is maintained distally displaced from the base element 2, whereby the strut arrangement 26 is longitudinally expanded so as to be movable within the surrounding arterial wall segment W.

As also shown in the figures, the handling member 30 comprises a radially protruding structure 38, in this case a ring-like shoulder, which serves to define an operational longitudinal position of the device.

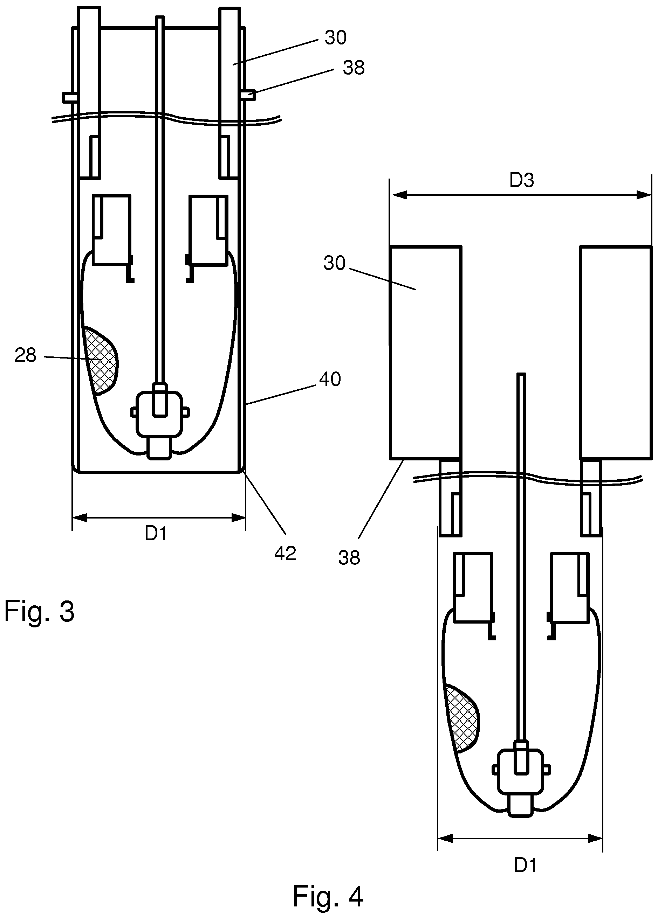

As furthermore shown in Fig. 3, the device comprises a longitudinally retractable sheath element 40 configured to surround the screen element 28 when the device is in its folded state. In order to provide easy passage through the aortic valve to be replaced, the distal edge region 42 of the sheath element is rounded.

In the movable state of the device shown in Fig. 1, the distal part below, i.e. distally from, the radially protruding structure has an outer diameter D1 which should not exceed about 5 mm. In the deployed state of the device shown in Fig. 2, the screen element 28 in its unfolded state has an outer diameter D2 of about 20 to 35 mm so as to form an anchoring against the surrounding wall segment W.

In the embodiment shown in Fig. 4, the elongated handling member 30 has a comparatively larger diameter D3, whereby the protruding structure 38 is formed by a distal face of the handling member.

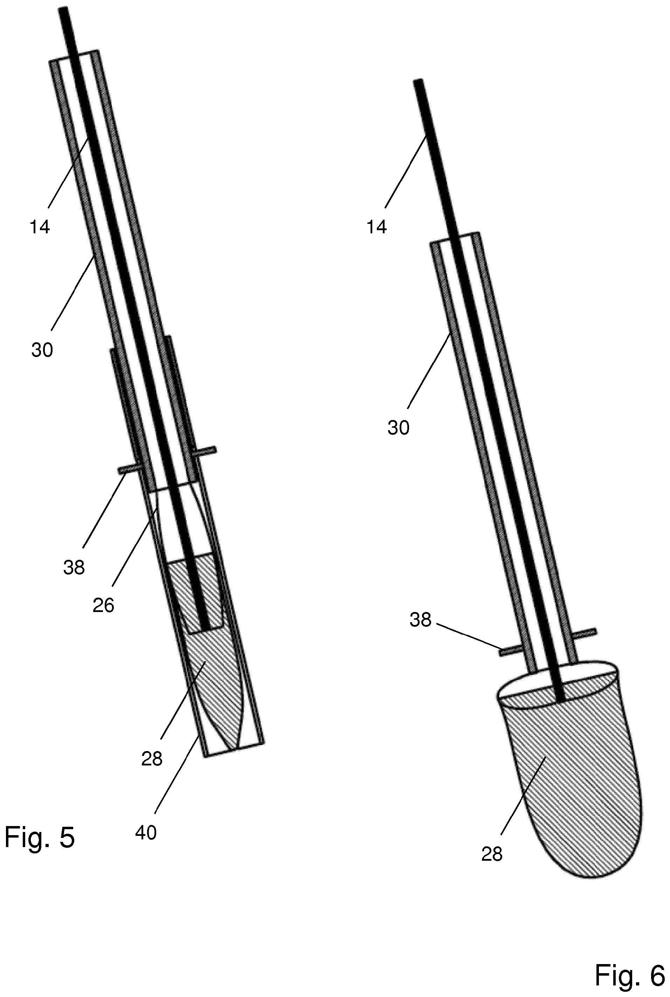

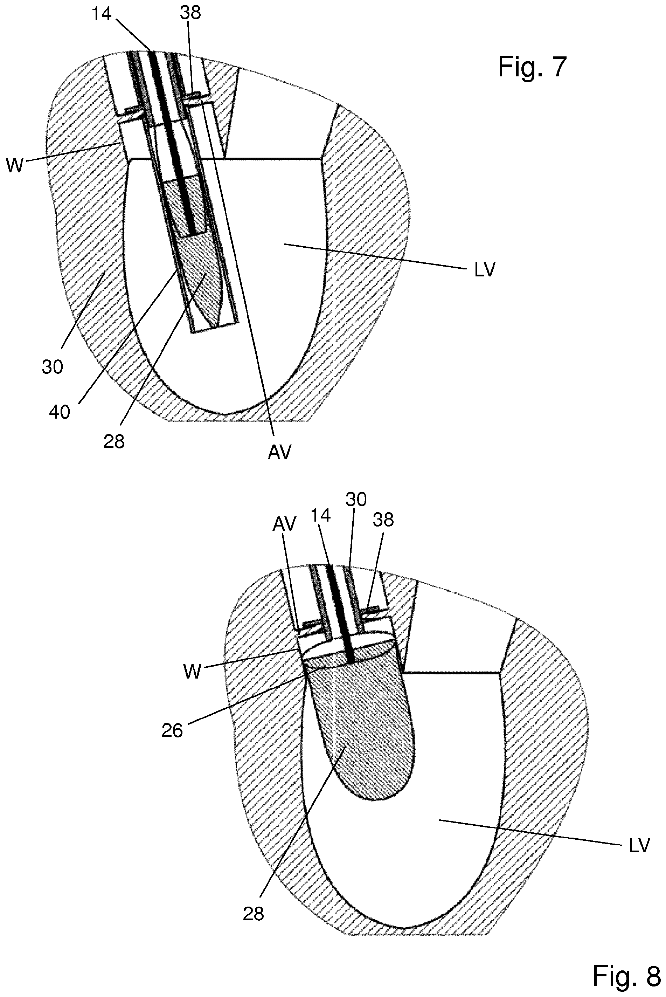

A further embodiment is illustrated in Figs. 5 to 8 in just schematic manner, first showing the protective device by itself and then showing a distal part of the device in situ. In particular, Figs. 5 and 7 show the device in its movable state, wherein the flexible strut arrangement 26 and the screen element 28 are contained within the sheath element 40. Figs. 6 and 8 show the device in its deployed state, after removal of the sheath element 40. The anatomic situation is shown in Figs. 7 and 8 including a schematic representation of the aortic valve AV, arterial wall segment W and left ventricular region LV.

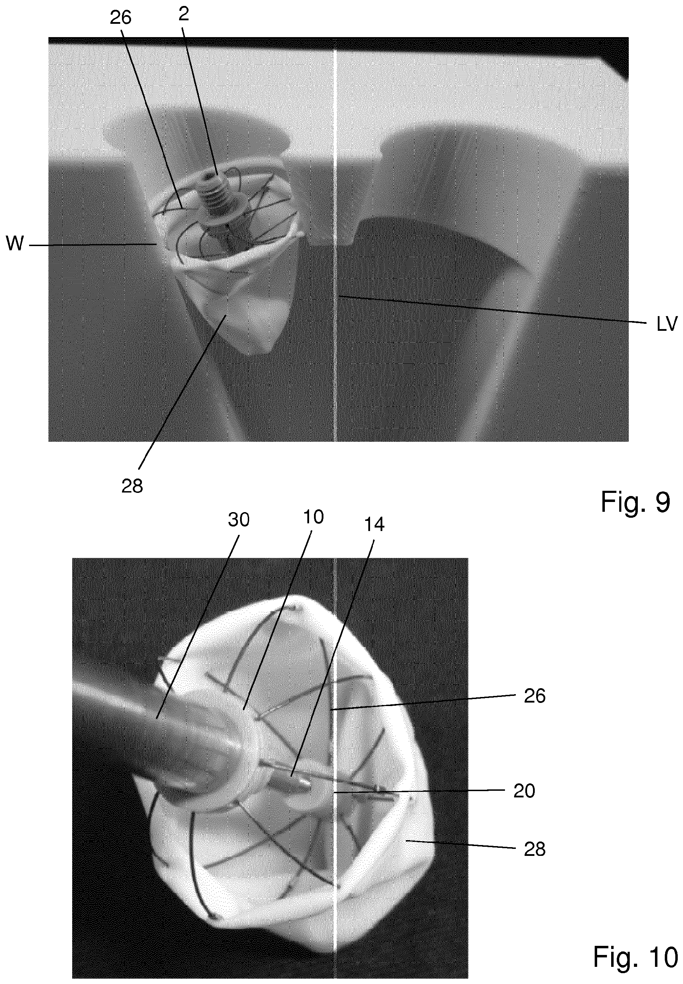

Further illustration of the operating principle is provided by Figs. 9 and 10. Fig. 9 shows a photographic model view of a distal part of a protective device in the deployed state after removal of the handling member. For comparison, Fig. 10 shows a photographic model view of a protective device in a transition state between movable and deployed states. In the situation shown, the handling member 30 is connected to the first coupling member 10, the elongated actuating member 14 is partially protruding in distal direction and connected to tip element 20.

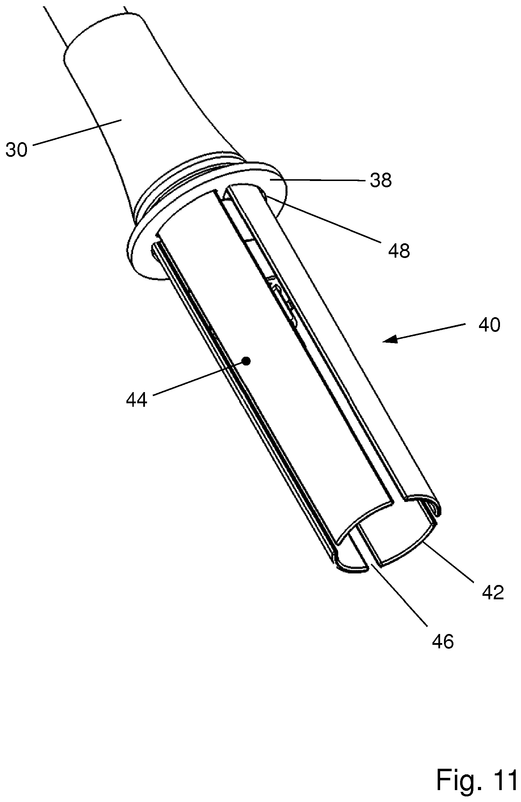

A fourth embodiment is illustrated in Figs. 11 to 14. Some components of the protective device in the movable state are shown in Fig. 11. These include the retractable sheath element 40 with rounded edge region 42, the handling member 30 and the ring-like protruding structure 38 attached thereto. The sheath element 40 comprises longitudinal ribs 44 separated by longitudinal slits 46 and in this manner is longitudinally retractable through the protruding structure 38 via passages 48 shaped as annulus sectors.

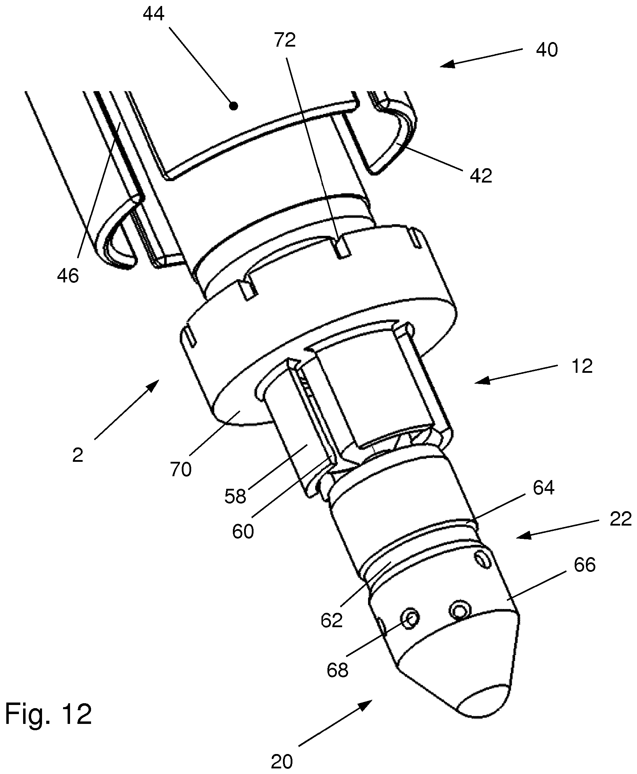

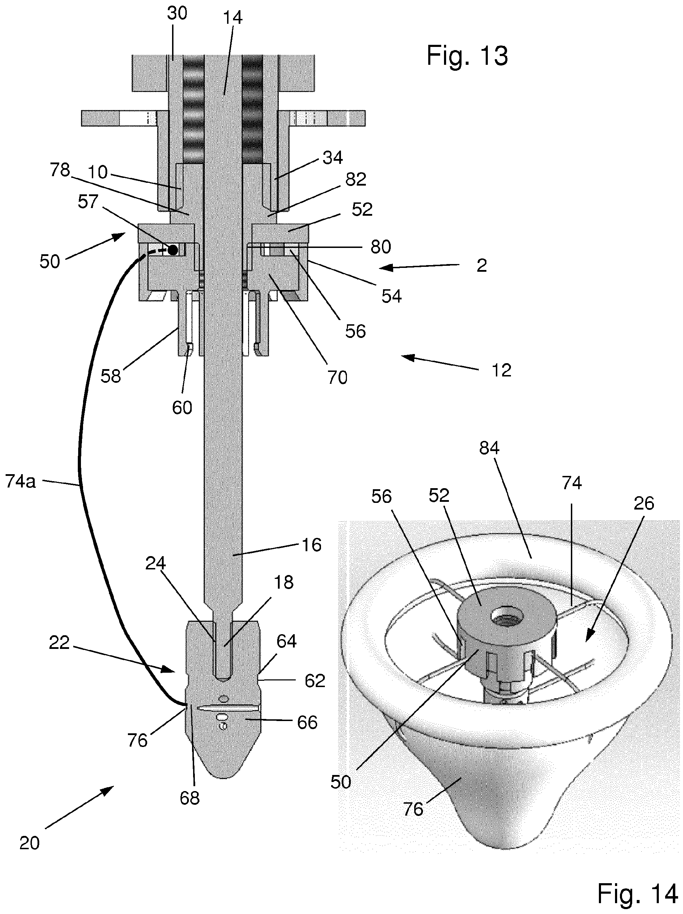

Figs. 12 and 13 show a distal portion of the device with partially retracted sheath element 40. The base element generally indicated as 2 comprises, at its distal end, the second coupling member 12. A covering ring 50, which is not shown in Fig. 12 for better clarity, has a proximal flange region 52 and a distally protruding cylindrical collar 54 with longitudinal slits 56 forming passages for axially sliding the covering ring over proximal strut ends 57. The second coupling member 12 comprises a plurality of cylindrical segments 58 with inward directed distal snapper ends 60. Also shown in Fig. 12 is the tip element 20 with fourth coupling member 22 configured as circumferential recess 62. In order to be releasably connectable with the second coupling member 12, the recess 62 is formed to engagingly receive the distal snapper ends 60 and to allow disengagement thereof by having an oblique transition zone 64. Moreover, the tip element 20 has a radial face portion 66 with connection sites 68 for distal strut ends.

The base element 2 is made up of several parts, including a distal base ring 70 having a face portion with a plurality of lateral connection passages 72 for receiving proximal ends 57 of resilient struts 74a. In Fig. 13 are shown distal strut ends 76 engaged in connection sites 68 of tip element 20. Base element 2 further comprises a proximal coupling ferrule 78 having a distal cylindrical part 80 insertable through the covering ring 50 and connectable to the distal base ring 70. The coupling ferrule 78 is provided at its proximal side with the first coupling member 10 and has a radially protruding section 82 for abutment against a proximal surface of the covering ring.

As shown in Fig. 14, the screen element 28 comprises an expandable ring element 84 connected to the strut arrangement 26 and forming a radially external portion of the screen element. In this example, the ring element 84 is configured as a cushion-like element ensuring safe anchoring of the device against a surrounding arterial wall region not shown in the drawing.

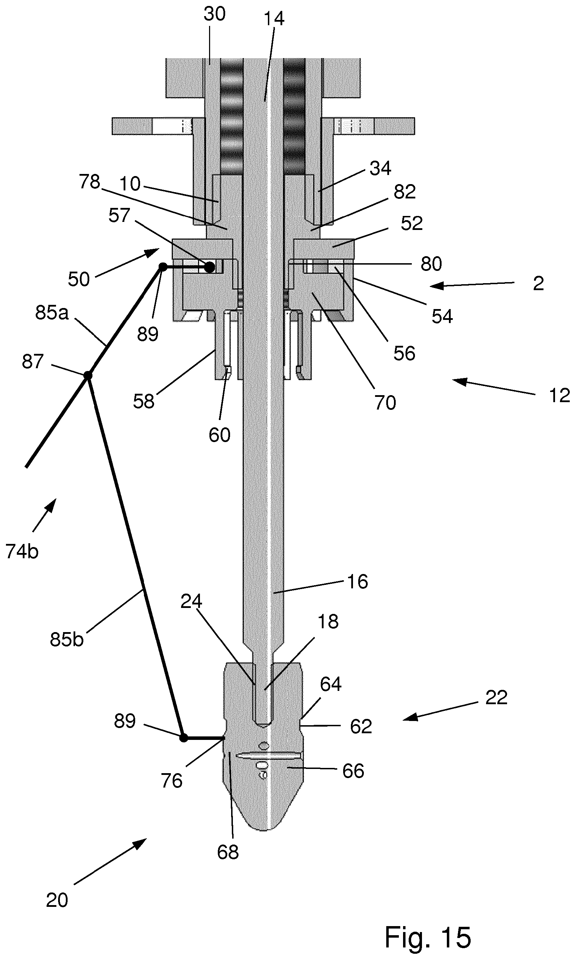

Another embodiment of the the distal part is shown in Fig. 15. In contrast to the arrangement shown in Fig. 13, the flexible strut arrangement is made up of articulated struts 74b. Each articulated strut comprises at least two strut segments 85a, 85b connected to each other by a sliding hinge member 87. Like in the case of Fig. 13, each articulated strut 74b has a proximal strut end 57 connected to a radial face portion of the base element 2 and a distal strut end 76 connected to a radial face portion 66 of the tip element 20.

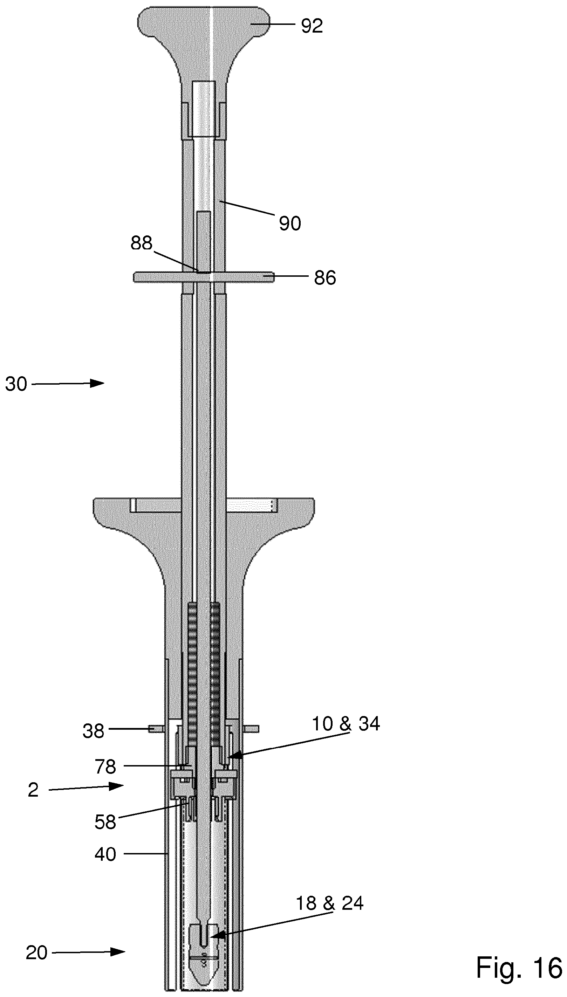

A fifth embodiment of a protective device is shown in Fig. 16. It is closely similar to the fourth embodiment, and only the most important differences will thus be described. The first pair of cooperating threads 10 and 34, and the second pair of cooperating threads 18 and 24, have identical sense of rotation. In other words, either the threads are all right-handed or they are all left-handed. Moreover, the elongated actuator member 14 and the elongated handling member 30 are rotationally coupled. For this purpose, a coupling pin 86 is inserted through a correspondingly dimensioned radial passage 88 in the elongated actuator member 14 and a correspondingly dimensioned radial passage 90 in the elongated handling member 30. In the example shown, the elongated handling member 30 is provided at its proximal end with a handling knob 92 rigidly connected thereto. By applying a rotational motion to handling knob 92, an operator can simultaneously rotate the handling member 30 and the actuator member 14, thereby either connecting or disconnecting both pairs of cooperating threads 10 & 34 and 18 & 24. Furthermore, the elongated actuator member 14 and the elongated handling member 30 are longitudinally decoupled. This allows compensation for different pitches of the two pairs of cooperating threads and any further relative longitudinal displacement between actuator member 14 and handling member 30. In the example shown, longitudinal decoupling is provided by having radial passage 90 formed as longitudinal slits, thus allowing coupling pin 86 and actuator member 14 to slide in longitudinal direction with respect to handling member 30.

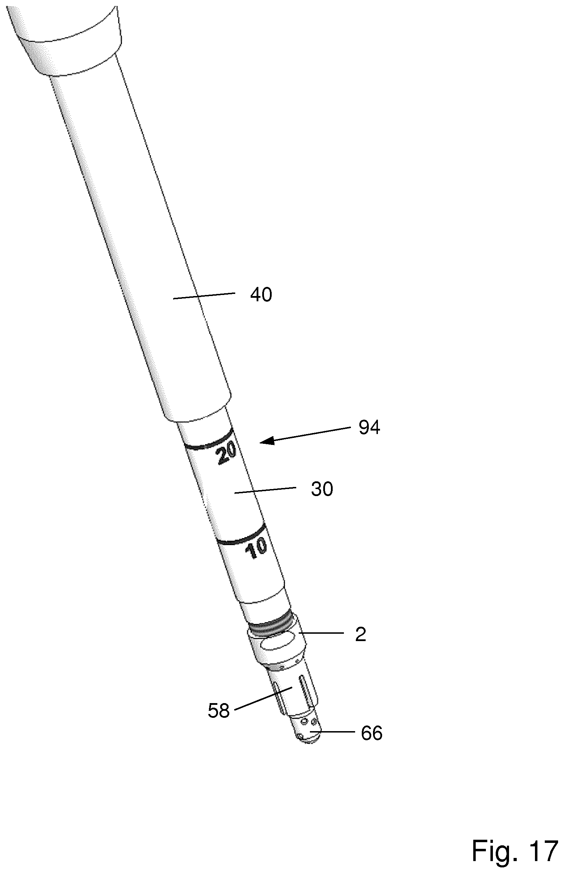

According to a sixth embodiment illustrated in Fig. 17, the device comprises a longitudinal marker scale 94 on the elongated handling member 30. Alternatively or additionally, such a marker scale can also be disposed on the sheath element.

In a further embodiment schematically illustrated in Fig. 2, the device comprises two string elements 96, only one of which is shown. Each string element is connected at its distal string end to a respective connection point 98 of the screen element 28. In the situation shown, the string element simply lies on the screen element. After removal of the elongated handling member 30, the string element can be grabbed at its distal end and pulled upwards past the base element 2 and secured by the operator for the case of later need.

### List or reference numerals

- 2: base element
- 4: proximal side of 2
- 6: distal side of 2
- 8: central passageway of 2
- 10: first coupling member of 2
- 12: second coupling member of 2
- 14: elongated actuator member
- 16: distal end of 14
- 18: third coupling member of 14
- 20: tip element
- 22: fourth coupling member of 20
- 24: fifth coupling member of 20
- 26: strut arrangement
- 28: screen element
- 30: elongated handling member
- 32: distal end of 30
- 34: sixth coupling member of 30
- 36: longitudinal passageway of 30
- 38: radially protruding structure of 30
- 40: sheath element
- 42: distal edge region of 40
- 44: longitudinal ribs of 40
- 46: longitudinal slits of 40
- 48: passages of 38 for 46
- 50: covering ring
- 52: proximal flange region of 50
- 54: protruding cylindrical collar of 50
- 56: longitudinal slits of 52
- 57: proximal strut ends of 74a, 74b
- 58: cylindrical segments of 12
- 60: snapper ends of 58
- 62: circumfrerential recess of 20
- 64: oblique transition zone of 62
- 66: radial face of 20
- 68: connection sites of 66
- 70: distal base ring of 2
- 72: lateral connection passages of 70
- 74a: resilient struts
- 74b: articulated struts
- 76: distal strut ends of 74
- 78: proximal coupling ferrule
- 80: distal cylindrical part of 78
- 82: radially protruding section of 78
- 84: expandable ring element
- 85a, 85b: strut segment of 74b
- 86: coupling pin
- 87: sliding hinge member of 74b
- 88: radial passage of 14
- 90: radial passage of 30
- 92: handling knob of 30
- 94: longitudinal marker scale
- 96: string element
- 98: connection point

## Claims

1. A protective device for open heart aortic valve replacement surgery in a patient, comprising:
- a base element (2) having a proximal side (4), a distal side (6) and a central passage (8) between the proximal side (4) and the distal side (6), the base element (2) further comprising, on its proximal side (4), a first coupling member (10) and, on its distal side (6), a second coupling member (12),
- an elongated actuator member (14) longitudinally slidable within the central passage (8) and having a distal end (16) provided with a third coupling member (18),
- a tip element (20) comprising:
- a fourth coupling member (22) releasably connectable with the second coupling member (12),
- a fifth coupling member (24) releasably connectable with the third coupling member (18),
- a flexible strut arrangement (26) forming a radially external connection between the base element (2) and the tip element (20),
- a screen element (28) surroundingly attached to the strut arrangement (26),
- an elongated handling member (30) having a distal end (32) provided with a sixth coupling member (34) releasably connectable with the first coupling member (10), the handling member (30) having a longitudinal passageway (36) configured to slidingly receive the actuator member (14),
- a longitudinally retractable sheath element (40) configured to surround the screen element (28) when the device is in its folded state;
wherein, in a deployed state of the device, the fourth coupling member (22) is connected with the second coupling member (12) and the strut arrangement (26) is radially expanded, thereby forming an anchoring support for attachment to a surrounding arterial wall segment (W) of the patient,
whereas, in a movable state of the device, the fourth coupling member (22) is released from the second coupling member (12) and the tip element (20) is maintained distally displaced from the base element (2), whereby the strut arrangement (26) is longitudinally expanded so as to be movable within the surrounding arterial wall segment (W) and through the aortic valve of the patient; where the flexible strut arrangement (26) comprises a plurality of resilient struts (74), each one having a proximal strut end (72) connected to a radial face portion of the base element (2) and a distal strut end (76) connected to a radial face portion (66) of the tip element (20) or
where the flexible strut arrangement (26) comprises a plurality of articulated struts (74b), each articulated strut comprising at least two strut segments (85a, 85b) connected to each other by a sliding hinge member (87), each articulated strut having a proximal strut end (57) connected to a radial face portion of the base element (2) and a distal strut end (76) connected to a radial face portion (66) of the tip element (20).

2. The protective device according to claim 1, wherein the screen element (28) is made of a non-porous foil or cloth.

3. The protective device according to claim 1 or 2, wherein the second coupling member (12) and the fourth coupling member (22) are configured as cooperating snap connections.

4. The protective device according to one of claims 1 to 3, wherein the first coupling member (10) and the sixth coupling member (34) are configured as first cooperating thread pair (10 & 34).

5. The protective device according to one of claims 1 to 4, wherein the third coupling member (18) and the fifth coupling member (24) are configured as second cooperating thread pair (18 & 24).

6. The protective device according to claims 4 and 5, wherein the first cooperating thread pair (10 & 34) and the second cooperating thread pair (18 & 24) have identical sense of rotation and wherein the elongated actuator member (14) and the elongated handling member (30) are rotationally coupled but longitudinally decoupled.

7. The protective device according to one of claims 1 to 6, wherein the handling member (30) comprises a radially protruding structure (38) configured to define an operational longitudinal position of the device.

8. The protective device according to one of claims 1 or 7, wherein the struts (74a, 74b) are connected to the base element (2) and to the tip element (20) in a hinged manner.

9. The protective device according to one of claims 1 to 8, wherein the base element (2) comprises:
- a distal base ring (70) provided with said radial face portion and said second coupling member (12), said face portion having a plurality of lateral connection passages (72) for receiving respective proximal strut ends (57),
- a covering ring (50) having a flange region (52) and a protruding cylindrical collar (54) provided with longitudinal slits (56) forming passages for axially sliding the covering ring over said proximal strut ends (72),
- a proximal coupling ferrule (78) having a distal cylindrical part (80) insertable through the covering ring (50) and connectable to the distal base ring (70), the coupling ferrule being provided with said first coupling member (10) and having a radially protruding section (82) for abutment against a proximal surface of the covering ring (50).

10. The protective device according to one of claims 1 to 9, wherein the screen element (28) comprises an expandable ring element (84) connected to the strut arrangement (26) and forming a radially external portion of the screen element.

11. The protective device according to one of claims 1 to 10, wherein, in the movable state of the device, a distal part thereof has an outer diameter (D1) not exceeding 5 mm.

12. The protective device according to one of claims 1 to 11, wherein, in the deployed state of the device, the screen element in its unfolded state has an outer diameter (D2) of 20 to 35 mm.

13. The protective device according to one of claims 1 to 12, further comprising a longitudinal marker scale (94) on the elongated handling member (30) and/or on the sheath element (40).

14. The protective device according to one of claims 1 to 13, further comprising at least two string elements (96), each one of said string elements being connected at its distal string end to a respective connection (98) of the screen element (28) and having a length sufficient for pulling a proximal end past the base element (2).

15. A kit for open heart aortic valve replacement surgery, comprising a protective device according to one of claims 1 to 14 in a sterilized packaged form.

## Patentansprüche

1. Schutzvorrichtung für offene Herzklappenersatzchirurgie bei einem Patienten, umfassend:
- ein Basiselement (2) mit einer proximalen Seite (4), einer distalen Seite (6) und einem zentralen Durchgang (8) zwischen der proximalen Seite (4) und der distalen Seite (6), wobei das Basiselement (2) weiter, an seiner proximalen Seite (4), ein erstes Kupplungselement (10) und, an seiner distalen Seite (6) ein zweites Kupplungselement (12) umfasst,
- ein längliches Betätigungselement (14), das in dem zentralen Durchgang (8) in Längsrichtung verschiebbar ist und ein distales Ende (16) aufweist, das mit einem dritten Kupplungselement (18) versehen ist,
- ein Spitzenelement (20), umfassend:
- ein viertes Kupplungselement (22), das lösbar mit dem zweiten Kupplungselement (12) verbunden werden kann,
- ein fünftes Kupplungselement (24), das lösbar mit dem dritten Kupplungselement (18) verbunden werden kann,
- eine flexible Strebenanordnung (26), die eine radial äußere Verbindung zwischen dem Basiselement (2) und dem Spitzenelement (20) bildet,
- ein Schutzelement (28), das um die Strebenanordnung (26) herum befestigt ist,
- ein längliches Handhabungselement (30) mit einem distalen Ende (32), das mit einem sechsten Kupplungselement (34) versehen ist, das lösbar mit dem ersten Kupplungselement (10) verbunden werden kann, wobei das Handhabungselement (30) einen länglichen Gang (36) aufweist, der eingerichtet ist, das Betätigungselement (14) verschiebbar aufzunehmen,
- ein längliches zurückziehbares Mantelelement (40), das eingerichtet ist, das Schutzelement (28) zu umgeben, wenn sich die Vorrichtung in ihrem gefalteten Zustand befindet;
wobei in einem eingesetzten Zustand der Vorrichtung das vierte Kupplungselement (22) mit dem zweiten Kupplungselement (12) verbunden ist und die Strebenanordnung (26) radial ausgedehnt ist, wodurch eine Verankerung zur Befestigung an einem umgebenden arteriellen Wandsegment (W) des Patienten gebildet wird,
während in einem beweglichen Zustand der Vorrichtung das vierte Kupplungselement (22) von dem zweiten Kupplungselement (12) gelöst ist und das Spitzenelement (20) distal verschoben zu dem Basiselement (2) gehalten wird, wodurch die Strebenanordnung (26) in Längsrichtung ausgedehnt ist, um so innerhalb des umgebenden arteriellen Wandsegments (W) und durch die Herzklappe des Patienten beweglich zu sein;
wobei die flexible Strebenanordnung (26) mehrere elastische Streben (74) umfasst, von welchen jede ein proximales Strebenende (72), das mit einem radialen Flächenabschnitt des Basiselements (2) verbunden ist, und ein distales Strebenende (76), das mit einem radialen Flächenabschnitt (66) des Spitzenelements (20) verbunden ist, aufweist, oder
wobei die flexible Strebenanordnung (26) mehrere gelenkige Streben (74b) umfasst, wobei jede gelenkige Strebe mindestens zwei Strebensegmente (85a, 85b) umfasst, die durch ein gleitendes Scharnierelement (87) miteinander verbunden sind, wobei jede gelenkige Strebe ein proximales Strebenende (57), das mit einem radialen Flächenabschnitt des Basiselements (2) verbunden ist, und ein distales Strebenende (76), das mit einem radialen Flächenabschnitt (66) des Spitzenelements (20) verbunden ist, aufweist.

2. Schutzvorrichtung nach Anspruch 1, wobei das Schutzelement (28) aus einer nicht porösen Folie oder einem Tuch hergestellt ist.

3. Schutzvorrichtung nach Anspruch 1 oder 2, wobei das zweite Kupplungselement (12) und das vierte Kupplungselement (22) als zusammenwirkende Schnappverbindungen eingerichtet sind.

4. Schutzvorrichtung nach einem der Ansprüche 1 bis 3, wobei das erste Kupplungselement (10) und das sechste Kupplungselement (34) als erstes zusammenwirkendes Gewindepaar (10 & 34) eingerichtet sind.

5. Schutzvorrichtung nach einem der Ansprüche 1 bis 4, wobei das dritte Kupplungselement (18) und das fünfte Kupplungselement (24) als zweites zusammenwirkendes Gewindepaar (18 & 24) eingerichtet sind.

6. Schutzvorrichtung nach den Ansprüchen 4 und 5, wobei das erste zusammenwirkende Gewindepaar (10 & 34) und das zweite zusammenwirkende Gewindepaar (18 & 24) identische Drehrichtungen haben und wobei das längliche Betätigungselement (14) und das längliche Handhabungselement (30) drehgekoppelt aber in Längsrichtung entkoppelt sind.

7. Schutzvorrichtung nach einem der Ansprüche 1 bis 6, wobei das Handhabungselement (30) eine radial vorstehende Struktur (38) umfasst, die eingerichtet ist, eine Betriebslängsposition der Vorrichtung zu definieren.

8. Schutzvorrichtung nach einem der Ansprüche 1 bis 7, wobei die Streben (74a, 74b) mit dem Basiselement (2) und dem Spitzenelement (20) gelenkig verbunden sind.

9. Schutzvorrichtung nach einem der Ansprüche 1 bis 8, wobei das Basiselement (2) umfasst:
- einen distalen Basisring (70), der mit dem radialen Flächenabschnitt und dem zweiten Kupplungselement (12) versehen ist, wobei der Flächenabschnitt mehrere seitliche Verbindungsdurchgänge (72) zum Aufnehmen entsprechender proximaler Strebenenden (57) aufweist,
- einen Abdeckring (50), der einen Flanschbereich (52) und einen vorstehenden zylindrischen Kranz (54) aufweist, der mit Längsschlitzen (56) versehen ist, die Durchgänge zum axialen Verschieben des Abdeckrings über die proximalen Strebenenden (72) bilden,
- eine proximale Kupplungshülse (78) mit einem distalen zylindrischen Teil (80), der durch den Abdeckring (50) einsetzbar ist und mit dem distalen Basisring (70) verbunden werden kann, wobei die Kupplungshülse mit dem ersten Kupplungselement (10) versehen ist und einen radial vorstehenden Abschnitt (82) zum Anliegen gegen eine proximale Oberfläche des Abdeckrings (50) aufweist.

10. Schutzvorrichtung nach einem der Ansprüche 1 bis 9, wobei das Schutzelement (28) ein ausdehnbares Ringelement (84) umfasst, das mit der Strebenanordnung (26) verbunden ist und einen radial externen Abschnitt des Schutzelements bildet.

11. Schutzvorrichtung nach einem der Ansprüche 1 bis 10, wobei im beweglichen Zustand der Vorrichtung ihr distaler Teil einen Außendurchmesser (D1) aufweist, der 5 mm nicht übersteigt.

12. Schutzvorrichtung nach einem der Ansprüche 1 bis 11, wobei im eingesetzten Zustand der Vorrichtung das Schutzelement in seinem entfalteten Zustand einen Außendurchmesser (D2) von 20 bis 35 mm aufweist.

13. Schutzvorrichtung nach einem der Ansprüche 1 bis 12, weiter umfassend eine Längsmarkierungsskala (94) auf dem länglichen Handhabungselement (30) und/oder auf dem Mantelelement (40).

14. Schutzvorrichtung nach einem der Ansprüche 1 bis 13, weiter umfassend mindestens zwei Fadenelemente (96), wobei jedes der Fadenelemente an seinem distalen Fadenende mit einer entsprechenden Verbindung (98) des Schutzelements (28) verbunden ist und eine ausreichende Länge aufweist, um ein proximales Ende über das Basiselement (2) hinaus zu ziehen.

15. Kit für offene Herzklappenersatzchirurgie, umfassend eine Schutzvorrichtung nach einem der Ansprüche 1 bis 14 in einer sterilisierten verpackten Form.

## Revendications

1. Dispositif protecteur pour chirurgie de remplacement de valvule aortique à cœur ouvert chez un patient, comprenant :
- un élément de base (2) ayant un côté proximal (4), un côté distal (6) et un passage central (8) entre le côté proximal (4) et le côté distal (6), l'élément de base (2) comprenant en outre, sur son côté proximal, un premier élément de couplage (10),et, sur son côté distal (6), un deuxième élément de couplage (12),
- un élément d'organe de commande allongé (14) coulissable longitudinalement dans le passage central (8) et ayant une extrémité distale (16) fournie avec un troisième élément de couplage (18) ;
- un élément de pointe (20) comprenant :
- un quatrième élément de couplage (22) connectable de manière relâchée avec le deuxième élément de couplage (12),
- un cinquième élément de couplage (24) connectable de manière relâchée avec le troisième élément de couplage (18),
- une disposition de support flexible (26) formant une connexion radialement externe entre l'élément de base (2) et l'élément de pointe (20),
- un élément d'écran (28) attaché en entourant la disposition de support (26),
- un élément de manipulation allongé (30) ayant une extrémité distale (32) fournie avec un sixième élément de couplage (34) connectable de manière relâchée avec le premier élément de couplage (10), l'élément de manipulation (30) ayant un passage longitudinal (36) configuré pour recevoir de manière coulissable l'élément d'organe de commande (14),
- un élément de gaine longitudinalement rétractable (40) configuré pour entourer l'élément d'écran (28) quand le dispositif est dans son état replié ;
dans un état déployé du dispositif, le quatrième élément de couplage (22) étant connecté avec le deuxième élément de couplage (12) et la disposition de support (26) étant expansée radialement, formant ainsi un support d'ancrage pour l'attachement à un segment de paroi environnant (W) du patient,
alors que, dans un état amovible du dispositif, le quatrième élément de couplage (22) est libéré du deuxième élément de couplage (12) et l'élément de pointe (20) est maintenu déplacé distalement de l'élément de base (2), moyennant quoi la disposition de support (26) est expansée longitudinalement pour être amovible dans le segment de paroi artérielle environnant (W) et à travers la valvule aortique du patient ;
où la disposition de support flexible (26) comprend une pluralité de supports résilients (74), chacun ayant une extrémité de support proximale (72) connectée à une portion de face radiale de l'élément de base (2) et une extrémité de support distale (76) connectée à une portion de face radiale (66) de l'élément de pointe (20) ou
où la disposition de support flexible (26) comprend une pluralité de supports articulés (74b), chaque support articulé comprenant au moins deux segments de support (85a, 85b) connectés l'un à l'autre par un élément de charnière coulissant (87), chaque support articulé ayant une extrémité de support proximale (57) connectée à une portion de face radiale de l'élément de base (2) et une extrémité de support distale (76) connectée à une portion de face radiale (66) de l'élément de pointe (20).

2. Dispositif protecteur selon la revendication 1, dans lequel l'élément d'écran (28) est constitué d'une feuille ou d'un vêtement non poreux.

3. Dispositif protecteur selon la revendication 1 ou 2, dans lequel le deuxième élément de couplage (12) et le quatrième élément de couplage (22) sont configurés comme connexions de clipsage coopérantes.

4. Dispositif selon l'une des revendications 1 à 3, dans lequel le premier élément de couplage (10) et le sixième élément de couplage (34) sont configurés comme une première paire de fils coopérante (10 & 34).

5. Dispositif selon l'une des revendications 1 à 4, dans lequel le troisième élément de couplage (18) et le cinquième élément de couplage (24) sont configurés comme seconde paire de fils coopérante (18 & 24).

6. Dispositif protecteur selon les revendications 4 et 5, dans lequel la première paire de fils coopérante (10 & 34) et la seconde paire de fils coopérante (18 & 24) ont un sens de rotation identique et l'élément d'organe de commande allongé (14) et l'élément de manipulation allongé (30) étant couplés rotationnellement mais découplés longitudinalement.

7. Dispositif protecteur selon l'une des revendications 1 à 6, dans lequel l'élément de manipulation (30) comprend une structure radialement en saillie (38) configurée pour définir une position longitudinale opérationnelle du dispositif.

8. Dispositif protecteur selon l'une des revendications 1 ou 7, dans lequel les supports (74a, 74b) sont connectés à l'élément de base (2) et à l'élément de pointe (20) de manière en charnière.

9. Dispositif protecteur selon l'une des revendications 1 à 8, dans lequel l'élément de base (2) comprend :
- un anneau de base distal (70) fourni avec ladite portion de face radiale et ledit deuxième élément de couplage (12), ladite portion de face ayant une pluralité de passages de connexion latéraux (72) pour recevoir les extrémités de support proximales respectives (57),
- un anneau de couverture (50) ayant une zone de bride (52) et un col cylindrique en saillie (54) fourni avec des fentes longitudinales (56) formant des passages pour faire coulisser axialement l'anneau de couverture sur lesdites extrémités de support proximales (72),
- une ferrule de couplage proximale (78) ayant une partie cylindrique distale (80) insérable à travers l'anneau de couverture (50) et connectable à l'anneau de base distal (70), la ferrule de couplage étant fournie avec ledit premier élément de couplage (10) et ayant une section radialement en saillie (82) pour l'ajouxtement contre une surface proximale de l'anneau de couverture (50).

10. Dispositif protecteur selon l'une des revendications 1 à 9, dans lequel l'élément d'écran (28) comprend un élément d'anneau expansible (84) connecté à la disposition de support (26) et formant une portion radialement externe de l'élément d'écran.

11. Dispositif protecteur selon l'une des revendications 1 à 10, dans lequel, dans l'état amovible du dispositif, une de ses parties distales a un diamètre externe (D1) ne dépassant pas 5 mm.

12. Dispositif protecteur selon l'une des revendications 1 à 11, dans lequel, dans l'état déployé du dispositif, l'élément d'écran dans son état non replié a un diamètre externe (D2) de 20 à 35 mm.

13. Dispositif protecteur selon l'une des revendications 1 à 12, comprenant en outre une échelle de marqueur longitudinale (94) sur l'élément de manipulation allongé (30) et/ou sur l'élément de gaine (40).

14. Dispositif protecteur selon l'une des revendications 1 à 13, comprenant en outre au moins deux éléments d'anneau (96), chacun desdits éléments d'anneau étant connecté à son extrémité d'anneau distale à une connexion respective (98) de l'élément d'écran (28) et ayant une longueur suffisante pour tirer une extrémité proximale après l'élément de base (2).

15. Kit pour chirurgie de remplacement de valvule aortique à cœur ouvert comprenant un dispositif protecteur selon l'une des revendications 1 à 14 sous une forme conditionnée stérilisée.
